# EUROPÄISCHE PATENTSCHRIFT

(11) **EP 2 872 063 B1**
(45) Veröffentlichungstag und Bekanntmachung des Hinweises auf die Patenterteilung: **17.08.2016**
(21) Anmeldenummer: 13744454.3
(22) Anmeldetag: 10.07.2013
(51) Int. Cl.: A61B 18/14, A61B 17/42, A61B 18/00

(54) **INSTRUMENT ZUM SCHNEIDEN VON KÖRPERGEWEBE**
INSTRUMENT FOR CUTTING BODY TISSUE
INSTRUMENT SERVANT À COUPER DES TISSUS CORPORELS

(30) Priorität: 12.07.2012 DE 102012013738
(43) Veröffentlichungstag der Anmeldung: 20.05.2015
(73) Patentinhaber: BOWA-electronic GmbH & Co. KG, 72810 Gomaringen (DE)
(72) Erfinder: GRAF, Thomas, 72810 Gomaringen (DE); DOPPELSTEIN, Alexander, 72411 Bodelshausen (DE); SPÜNTRUP, Carolin, 66119 Saarbrücken (DE); NOÉ, Karl-Günter, 50769 Köln (DE)
(74) Vertreter: Schneider, Peter Christian
(86) Internationale Anmeldenummer: PCT/EP2013/064593
(87) Internationale Veröffentlichungsnummer: WO 2014/009420

(56) Entgegenhaltungen:
- US-A1- 2004 002 701
- US-A1- 2006 094 983
- US-A1- 2012 143 209
- US-B2- 6 641 581

## Beschreibung

### Gebiet der Erfindung

Die Erfindung betrifft ein Instrument zum Schneiden von Körpergewebe umfassend
- einen Instrumentenschaft,
- eine dem distalen Ende des Instrumentenschaftes benachbart angeordnete, gegenüber einer Längsachse des Schaftes ausklappbare und um die Längsachse drehbare Schneide, und
- ein am proximalen Ende des Instrumentenschaftes angeordnetes Bedienteil.

### Stand der Technik

Aus Banerjee C., Kaiser N., Hatzmann W., Reiss G., Schmitz J., Hellmich M., Noé G. "Reduktion der Spottingrate nach laparoskopischer suprazervikaler Hysterektomie" Geburtsh Frauenheilk 2010; 70: 798-802 ist es bekannt, den Zervikalkanal endoskopisch tief konusförmig auszuschneiden und den verbleibenden Zervikalkanalanteil zusätzlich mit Strom zu veröden. Durch das konusförmig endoskopische Aushöhlen des Zervixstumpfes können endometroide Zellen im isthmischen und zervikalen Bereich besser erfasst werden als beim alleinigen Veröden. Dabei weist ein suprapubischer Zugang zum Zervikalkanal über einen Trokar bzw. eine Trokarhülse gegenüber einem vaginalen Zugang zum Zervikalkanal gewisse Vorteile, beispielsweise eine geringe Spottingrate, auf.

Aus der US 2012/0143209 A1 (Basis für den Oberbegriff des Anspruchs 1) ist ein medizinisches Instrument für eine laparoskopische suprazervikale Hysterektomie (LASH) bekannt. Das bekannte Instrument zum Schneiden von Körpergewebe umfasst dabei einen Instrumentenschaft, eine dem distalen Enden des Instrumentenschaftes benachbart angeordnete und gegenüber einer Längsachse des Schaftes ausklappbare bzw. ausschwenkbare und um die Längsachse drehbare Schneide sowie ein am proximalen Ende des Instrumentenschaftes angeordnetes Bedienteil.

Nachteilig dabei ist, dass dieses Instrument nicht für einen suprapubischen Zugang über einen bekannten Laparoskoptrokar bzw. eine Trokarhülse zum Zervixkanal geeignet ist. Weiterhin ist aus der US 2006/09490083 A1 ein an einem distalen Schaftende angeordneter Gewebeanschlag bekannt, der als ein vier hebeliges Parallelogramm ausgebildet ist, das um eine quer zur Längsachse angeordnete Achse seitlich so ausschwenkbar ist, dass jeweils zwei der Hebel quer zur Längsachse seitlich ausklappbar sind.

Aus der US 6 641 581 B2 ist ein Instrument zum Schneiden von Körpergewebe bekannt, das einen Instrumentenschaft aufweist, an dessen distalem Ende ein längsverschieblich und rotierbarer Elektrodenträger herausragt, an dessen distalem Ende seitlich ein gebogener Arm seitlich abstehend angeordnet ist, der eine Schneidelektrode aufspannt.

Nachteilig dabei ist, dass das distale Ende des Instrumentenschaftes bzw. des Elektrodenträgers wegen des starren seitlich herausragenden Armes nicht geeignet ist, durch eine Trokarhülse in eine Körperhöhle mit zu schneidendem Gewebe eingeführt zu werden. Weiterhin nachteilig ist, dass dieses Instrument keinen Gewebeanschlag aufweist.

Aus der US 2004/002701 A1 ist ein Schaftende mit zwei seitlich ausklappbaren Backen bekannt. Diese Backen sind als ein Ultraschallgerät ausgebildet, dass in die distale Richtung strahlt. Insofern würde der Fachmann die Ersetzung des mechanischen Gewebeanschlages aus der D1 mit aus der D4 bekannten Backen nicht als übliche Vorgehensweise ansehen.

### Aufgabenstellung

Aufgabe der vorliegenden Erfindung ist es, ein Instrument zur Verfügung zu stellen, das insbesondere geeignet ist, den Gebärmutterhals konusförmig auszuhöhlen und mit dem ein suprapubischer Zugang zum Gebärmutterhals über eine Laparoskop- bzw. Trokarhülse erfolgen kann.

### Darlegung der Erfindung

Diese Aufgabe wird in Verbindung mit dem Oberbegriff des Anspruches 1 dadurch gelöst, dass am distalen Ende des Instrumentenschaftes eine um die Längsachse drehbare Außenhülse angeordnet ist, die quer zu ihrer Längsrichtung eine seitliche Öffnung aufweist, aus der die Schneide ausschwenkbar ist.

Durch die seitlich ausschwenkbare bzw. ausklappbare (ausschwenkbar und ausklappbar werden im Folgendem synonym benutzt) Schneide, die beispielsweise als eine Art Messer oder als eine (Hochfrequenz-) Schneidelektrode ausgebildet sein kann, und den in eine Wirkposition bringbaren Gewebeanschlag mit einem vorgegebenen fixen Abstand zur Anlenkstelle kann in einer nicht in Wirkposition, beispielsweise bei nicht ausgeklapptem Gewebeanschlag, befindlichen Grundstellung der Instrumentenschaft, d.h. sein distales Ende, problemlos über eine geeignete übliche Trokarhülse in eine Körperhöhle mit zu schneidendem Gewebe, beispielsweise einen Zervixkanal, eingeführt werden. Dabei lässt sich der Gewebeanschlag beispielsweise seitlich in seine Wirkposition ausklappen und das Instrument in seiner Position fixieren.

Durch den Gewebeanschlag lässt sich somit die vorgesehene Schneidposition der Schneide Fixieren bzw. Sicherstellen.

Die Schneide lässt sich aus der seitlichen Öffnung der drehbaren Außenhülse in eine vorgesehene Stellung herausklappen bzw. herausschwenken und durch Rotation der Schneide um die Längsachse des Instrumentenschaftes drehen, so dass das umliegende Gewebe kegelförmig ausgeschnitten wird.

Gemäß einer bevorzugten Ausführungsform der Erfindung beträgt der fixe Abstand zwischen der Anschlagfläche des Gewebeanschlages in seiner Wirkposition (beispielsweise an der Portiowand) zur Anlenkstelle der Schneide mehr als 1,5 mm. Dadurch lässt sich beispielsweise bei der sogenannten LASH-Konisation ein sicheres Abtragen der Mucosa im Zervixkanal gewährleisten. Bewährt hat sich insbesondere ein fixer Abstand von etwa 4 bis 20 mm und bevorzugt 12 mm. Zur Anpassung an anatomische Gegebenheiten sind auch größere oder kleinere Abstände möglich.

Nach einer weiteren bevorzugten Ausführungsform der Erfindung ist der Gewebeanschlag um eine am distalen Ende des Instrumentenschaftes angeordnete erste Querachse in seine Wirkposition ausklappbar. Der Gewebeanschlag kann dabei aus einer in Längsrichtung angeordneten Grundstellung in seine Wirkposition aus der Längsachse herausgeklappt werden. In der Wirkposition kann die rückwärtige, d. h. proximal gerichtete Anschlagfläche gegenüber der Längsachse einen Winkel von bevorzugt 90 Grad aufweisen. Aber auch Zwischenstellungen und Winkel über 90 Grad sind möglich.

Gemäß einer bevorzugten Ausführungsform der Erfindung weist die Schneide an mindestens einer ihrer beiden parallel zur Längsachse verlaufenden Längsseiten eine Schneidkante auf.

Durch die scharfe Schneidkante bildet die Schneide ein um die Längsachse drehbares Messer, mit dem sich ein Gewebekegel mechanisch ausschneiden lässt. Mit Hilfe einer Koagulationselektrode oder durch eine Laserbestrahlung kann die beschnittene Gewebeoberfläche koaguliert werden.

Nach einer weiteren bevorzugten Ausführungsform der Erfindung weist der Instrumentenschaft ein Außenschaftrohr auf, dessen distales Ende mit der Außenhülse verbunden ist, und wobei in dem Außenschaftrohr ein Innenschaftrohr angeordnet ist. Zwischen Außenhülsen und Innenschaftrohr ist eine an ihrer proximalen Stirnseite die Schneide aufweisende Innenhülse angeordnet. Die Innenhülse ist dabei zusammen mit der Außenhülse und dem Außenschaftrohr um das Innenschaftrohr drehbar. Zudem ist das Außenschaftrohr mit der Außenhülse gegenüber dem Innenschaftrohr und der Innenhülse längsverschieblich ausgebildet.

Soweit die Schneide an ihrem distal angelenkten Ende federnd ausgebildet ist, kann durch Verschieben des Außenschaftrohres mit der Außenhülse die seitliche Öffnung der Außenhülse mit dem proximalen Ende der Schneide in Deckung gebracht werden, so dass deren proximales Ende aufgrund der Federwirkung aus der seitlichen Öffnung herausklappt bzw. herausschwenkt. Durch Zurückziehen des Außenschaftrohres mit der Außenhülse wird das angelenkte Ende der Schneide abgedeckt und die Schneide in die Öffnung wieder eingeklappt. Das proximale Ende der Schneide ist bevorzugt als ein freies Ende ausgebildet, kann grundsätzlich aber auch mit einer längsverschieblichen Gelenkanordnung oder Ähnlichem verbunden sein.

Gemäß einer weiteren bevorzugten Ausführungsform der Erfindung sind die Innenhülse und die Schneide mindestens im Bereich ihrer Anlenkstelle aus einer Formgedächtnislegierung ausgebildet. Dabei ist die Formgedächtnislegierung so ausgelegt, dass eine vorgegebene maximale ausgeklappte Stellung die Vorzugslage darstellt, wobei das freie Ende der Schneide durch Abdecken mit der Außenhülse "eingeklappt" wird und bei Freigabe das freie Ende in seine vorgegebene Ausklappstellung ausklappt.

Nach einer weiteren bevorzugten Ausführungsform der Erfindung ist am Bedienteil ein längsverschiebliches erstes Bedienelement angeordnet, wobei das Außenschaftrohr mit der Außenhülse zum Betätigen der Schneide, d.h. zum aus- und einklappen der Schneide, verschiebbar ist. Das Bedienelement ist dabei in unterschiedlichen Raststellungen verrastbar. Durch die Verrastung des ersten Bedienelementes lässt sich die Schneide zwischen einer eingeklappten und maximal ausgeklappten Stellung in verschiedenen Winkeln gegenüber der Längsachse fixieren.

Nach einer weiteren Ausführungsform der Erfindung ist am Bedienteil ein zweites drehbares Bedienelement angeordnet, mit dem das Außenschaftrohr mit der aus der Außenhülse ausgeklappten Schneide um die Längsachse des Instrumentenschaftes drehbar ist. Durch Drehen der Schneide um die Längsachse kann somit auf einfache Weise ein kegel- bzw. konusförmiger Gewebekegel ausgeschnitten werden.

Gemäß einer weiteren bevorzugten Ausführungsform der Erfindung ist die Schneide als eine Elektrode ausgebildet. Durch Verbinden der Schneide mit einem aktiven Pol eines Hochfrequenzgenerators, wobei der Patient mit einer großflächigen Neutralelektrode verbunden wird, kann mit der Schneide ein elektrochirurgischer Schnitt gelegt werden. Über die flache Seite der Schneide lässt sich zudem das kontaktierte Gewebe koagulieren.

Anstelle einer großflächigen Neutralelektrode kann auch der Gewebeanschlag als eine Elektrode ausgebildet sein, die mit dem Hochfrequenzgenerator verbunden ist, wobei die Schneide und der Gewebeanschlag ein bipolares Elektrodenpaar bilden.

Nach einer weiteren bevorzugten Ausführungsform der Erfindung weist der Gewebeanschlag am distalen Ende des Instrumentenschaftes zwei in einer u-förmigen, d.h. gabelförmigen Aufnahme angeordnete Backen mit spiegelsymmetrisch ausgebildeten Führungsnuten auf. Dabei sind die Backen mit ihren Führungsnuten entgegengesetzt zueinander um eine quer zur Längsachse in der Aufnahme angeordnete erste Querachse verschwenkbar, wobei die Backen an ihren proximalen Enden über eine parallel zur ersten Querachse angeordnete zweite Querachse mit einem distalen Ende einer in dem Innenschaftrohr längsverschieblich geführten Hubstange verbunden sind. Durch eine Längsverschiebung der Hubstange in distaler Richtung wird somit die zweite Querachse in Richtung der fixen ersten Querachse bewegt, so dass sich die Backen ähnlich einem Zangenmaul in radialer Richtung nach auswärts aufklappen und durch Verschiebung der Hubstange in proximaler Richtung schließen.

Nach einer weiteren bevorzugten Ausführungsform der Erfindung steht die Hubstange an ihrem dem distalen Ende abgewandten proximalen Ende im Bedienteil mit einem dritten Bedienelement in einer Wirkverbindung. Dabei ist der Gewebeanschlag über das dritte Bedienelement in unterschiedlichen Raststellungen verrastbar.

Das erfindungsgemäße Instrument zum Schneiden von Körpergewebe ist insbesondere als laparoskopisches Instrument zum Schneiden und/oder Koagulieren der inneren Portio geeignet.

Das Instrument lässt sich bei eingeklappter Schneide und eingeklappten Gewebeanschlag, also in einer Grundstellung, über eine Trokarhülse in den Zervixkanal und weiter durch die Portio einführen. Der Gewebeanschlag lässt sich ausklappen und gegen die Portio ziehen. Nach Ausklappen der Schneide und Aktivieren des Stromes erfolgt ein Schneiden unter Drehung des Außenschaftrohres zusammen mit der Außenhülse und der Innenhülse, wobei der Gewebeanschlag mit dem Innenschaftrohr sich nicht mitdreht. Nach Einklappen/Einschwenken der Schneide und des Gewebeanschlages kann das Instrument aus der Trokarhülse herausgezogen werden. Die Schneide lässt sich beispielsweise um einen Winkel zwischen 0 und 45° ausschwenken. Dadurch ist eine präzise Einstellbarkeit des Gewebeabtrages gegeben. Die präzise Positionierung wird durch den rückseitigen Anschlag des Gewebeanschlages am Portio-Eingang unterstützt.

### Kurzbeschreibung der Zeichnungen

Es zeigen:

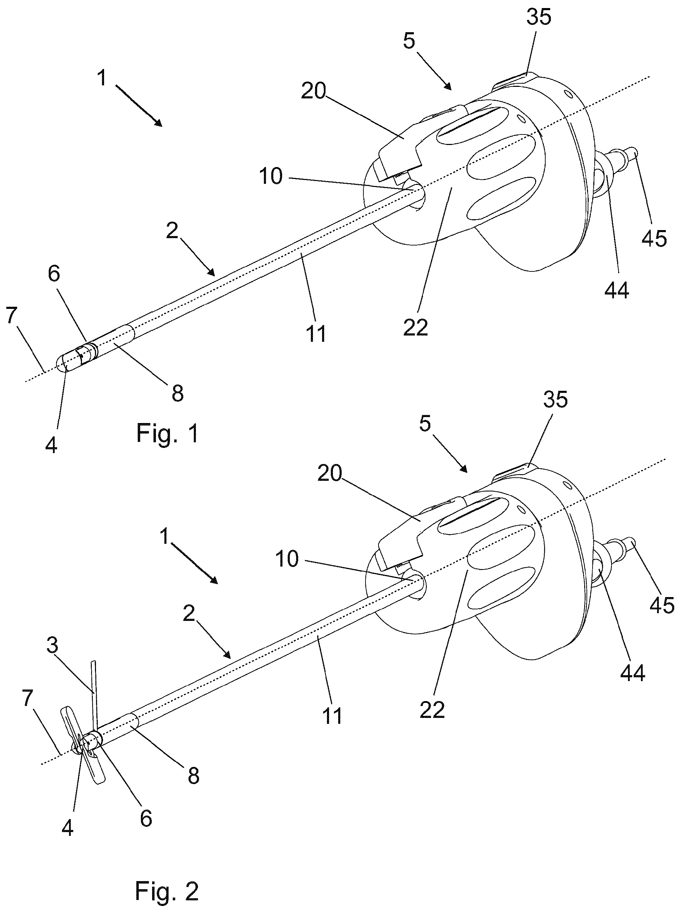

- Figur 1:: eine räumliche Darstellung eines Instrumentes zum Schneiden von Körpergewebe mit eingeschwenkter Schneide und eingeklapptem Gewebeanschlag, d.h. Schneide und Gewebeanschlag befinden sich in einer Grundstellung;
- Figur 2:: eine räumliche Darstellung des Instrumentes von Figur 1 mit ausgeschwenkter Schneide und ausgeklapptem Gewebeanschlag;

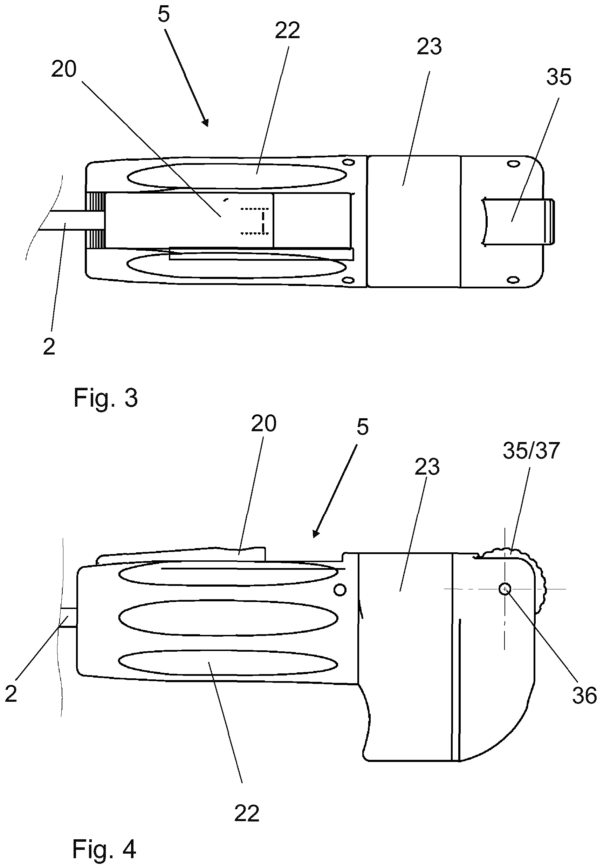

- Figur 3:: eine Draufsicht auf das Bedienteil von Figur 1;
- Figur 4:: eine Seitenansicht des Bedienteils von Figur 3;

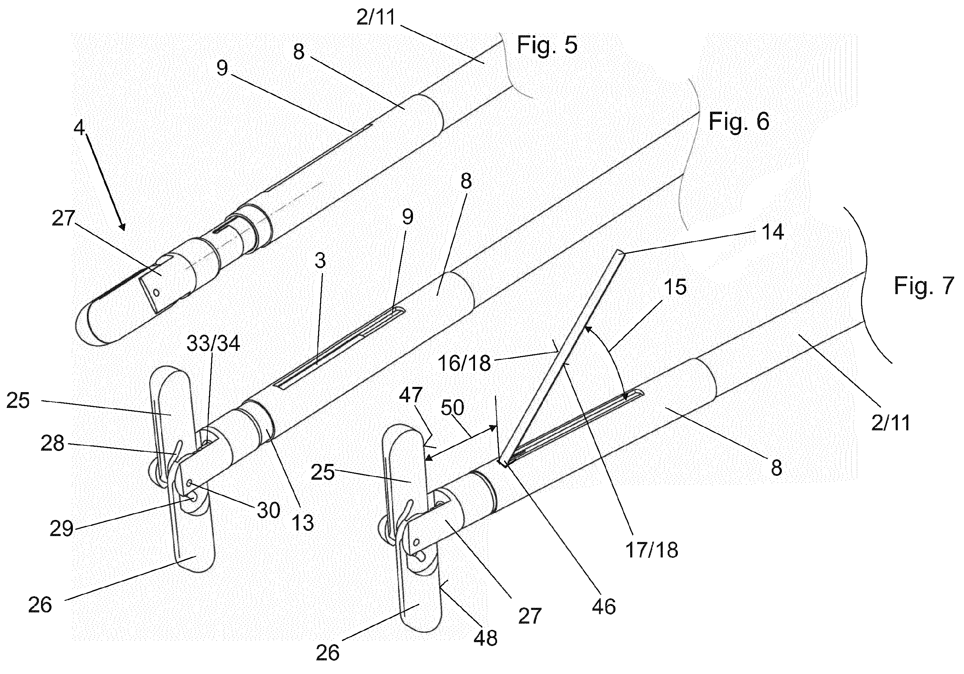

- Figur 5:: eine räumliche Darstellung des distalen Endes von Figur 1 mit eingeklapptem Gewebeanschlag (Grundstellung) und eingeschwenkter Schneide;
- Figur 6:: eine räumliche Darstellung des distalen Endes von Figur 5 mit ausgeklapptem Gewebeanschlag (Wirkposition) und eingeschwenkter Schneide;
- Figur 7:: eine räumliche Darstellung des distalen Endes von Figur 5 mit ausgeklapptem Gewebeanschlag (Wirkposition) und ausgeschwenkter Schneide;

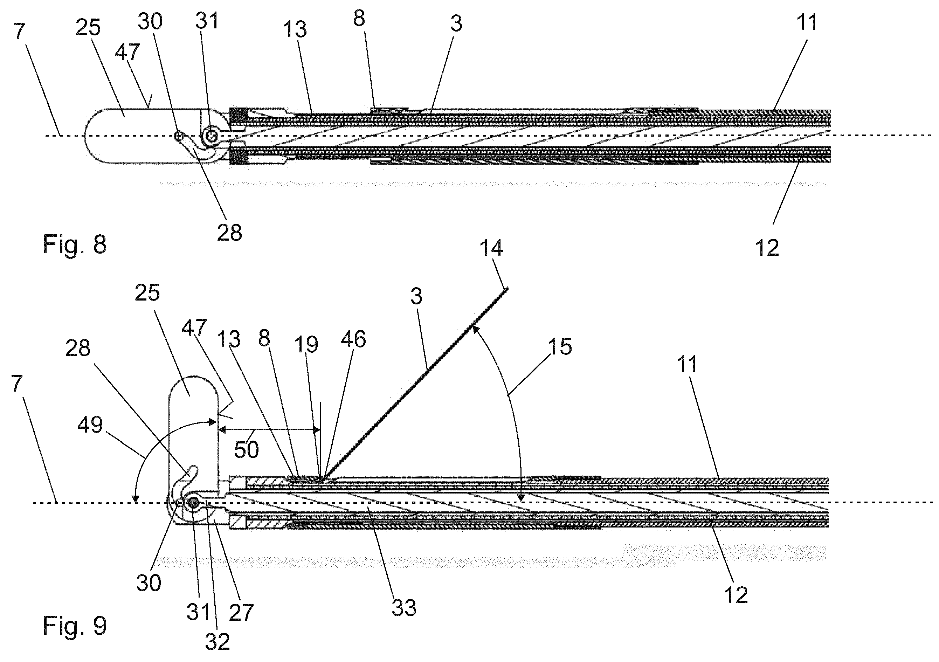

- Figur 8:: eine Seitenansicht im Schnitt des distalen Endes von Figur 5;
- Figur 9:: eine Seitenansicht im Schnitt des distalen Endes von Figur 7;

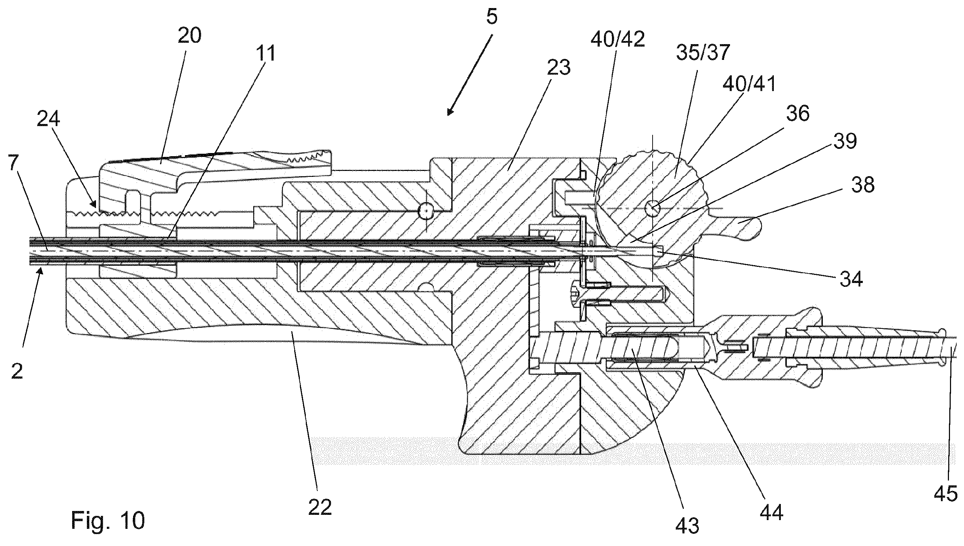

- Figur 10:: eine Seitenansicht des Bedienteils von Figur 1 im Schnitt und vergrößerter Darstellung.

### Beschreibung bevorzugter Ausführungsformen

Gleiche Bezugszeichen in den Figuren deuten auf gleiche oder analoge Bauteile hin. Die nachfolgende Beschreibung bezieht sich, sofern kein ausschließlicher Bezug auf eine spezielle Figur gegeben ist, auf sämtliche Figuren.

Ein Instrument 1 zum Schneiden von Körpergewebe besteht im Wesentlichen aus einem Instrumentenschaft 2, einer Schneide 3, einem Gewebeanschlag 4 und einem Bedienteil 5.

An einem dem Operateur abgewandten distalen Ende 6 des Instrumentenschaftes 2 ist eine um eine Längsachse 7 des Instrumentenschaftes 2 drehbare Außenhülse 8 angeordnet, die quer zu ihrer Längsachse eine seitliche Öffnung, d.h. einen rechtwinkligen Durchbruch, also eine Art Fenster, aufweist. Zum Operateur hin weist der Instrumentenschaft 2 an seinem proximalen Ende 10 das Bedienteil 5 auf. Der Instrumentenschaft 2 besteht aus einem Außenschaftrohr 11 und einem in dem Außenschaftrohr 11 beweglich (längsverschieblich und drehbar) geführten Innenschaftrohr 12.

Das distale Ende 6 des Außenschaftrohres 11 ist mit dem proximalen Ende 6 der Außenhülse 8 über ein Gewinde für Reinigungszwecke lösbar verbunden. Zwischen der Außenhülse 8 und dem Innenschaftrohr 12 ist eine Innenhülse 13 angeordnet, die an ihrer proximalen Stirnseite in die Schneide 3 übergeht. Soweit die seitliche Öffnung 9 mit der Schneide 3 in Deckung gebracht wird, klappt die Schneide 3, die mit ihrem distalen Ende an der Innenhülse 13 angelenkt ist, mit ihrem in proximaler Richtung freien Ende 14 durch die seitliche Öffnung 9 hindurch und bildet gegenüber der Längsachse 7 einen maximalen Ausschwenkwinkel 15 von beispielsweise 45°.

Die Schneide 3 weist an ihren beiden parallel zur Längsachse 7 verlaufenden Längsseiten 16, 17 eine Schneidkante 18 auf. Die Innenhülse 13 und die mit ihr verbundene Schneide 3 sind aus einer Formgedächtnislegierung ausgebildet, wobei der Anlenkstelle 19 der maximale Ausschwenkwinkel 15 eingeprägt ist. Formgedächtnislegierungen (shape memory alloy, SMA) die auch als Memory-Metalle bezeichnet werden, sind dem Fachmann bekannt. Diese weisen das Phänomen auf, dass sie sich an eine frühere Formgebung trotz nachfolgender starker Verformung scheinbar "erinnern" können. Als Werkstoffe kommen infrage:
- NiTi (Nickel-Titan; Nitinol)
- CuZn (Kupfer-Zink)
- CuZnAl (Kupfer-Zink-Aluminium)
- CuAlNi (Kupfer-Aluminium-Nickel)
- FeNiAl (Eisen-Nickel-Alumnium).

Die Innenhülse 13 ist zusammen mit der Außenhülse 8 und dem Außenschaftrohr 11 um das Innenschaftrohr 12 drehbar. Das Außenschaftrohr 11 mit der Außenhülse 8 ist gegenüber dem Innenschaftrohr 12 und der Innenhülse 13 längsverschieblich ausgebildet. Durch die Längsverschiebung zwischen Außenhülse 8 und Innenhülse 13 folgt das ein- bzw. Ausklappen der Schneide 3 aus der seitlichen Öffnung 9 der Außenhülse 8.

Über ein am Bedienteil 5 angeordnetes längsverschiebliches erstes Bedienelement 20, das mit dem proximalen Ende 21 des Außenschaftrohres 11 verbunden ist, lässt sich die Außenhülse 8 gegenüber der Innenhülse 13 mit der Schneide 3 in Längsrichtung verschieben, so dass die Schneide 3 an ihrer Anlenkstelle 19 abgedeckt und damit eingeklappt/eingeschwenkt wird oder freigegeben und damit ausgeklappt/ausgeschwenkt wird.

Das Bedienteil 5 weist ein zweites Bedienelement 22 auf, das um die Längsachse 7 drehbar an einem mit dem Innenschaftrohr 12 fest verbundenen Hauptkörper 23 des Bedienteils 5 gelagert ist. In dem zweiten Bedienelement 22 des Bedienteiles 5 ist das erste Bedienelement 20 längsverschieblich und über eine Rastvorrichtung 24 in unterschiedlichen Raststellungen verrastbar. Durch Drehen des zweiten Bedienelementes 22 lässt sich das Außenschaftrohr 11 mit Außenhülse 8 und dem ersten Bedienelement 20 um das Innenschaftrohr 12 drehen. Wird das erste Bedienelement 20 in proximaler Richtung zurückgezogen, wird die Schneide 3 eingeklappt. Wird das erste Bedienelement 20 in distaler Richtung verschoben, wird die Schneide, je nach Deckungsgrad der seitlichen Öffnung mit der Schneide ausgeklappt/ausgeschwenkt. Unterschiedliche Raststellungen des ersten Bedienelementes 20 führen somit zu unterschiedlichen Ausschwenkwinkeln 15 der Schneide 3.

In Verlängerung des Instrumentenschaftes 2 ist an dessen distalem Ende 6 der Gewebeanschlag 4 angeordnet. Der Gewebeanschlag 4 besteht aus zwei entgegengesetzt zueinander verschwenkbaren Backen 25, 26, die in einer u-förmigen Aufnahme 27 angeordnet sind. Die Backen 25, 26 weisen jeweils eine Führungsnut 28, 29 auf. Die beiden Führungsnuten 28, 29 sind spiegelsymmetrisch zueinander ausgebildet. Die Backen 25, 26 mit ihren Führungsnuten 28, 29 sind damit entgegengesetzt zueinander um eine quer zur Längsachse 7 in der Aufnahme 27 angeordnete erste Querachse 30 verschwenkbar. Dabei sind die Backen 25, 26 an ihren proximalen Enden über eine zweite Querachse 31 mit einem distalen Ende 32 einer in dem Innenschaftrohr 12 längsverschieblich geführten Hubstange 33 verbunden. Die Hubstange steht an ihrem proximalen Ende 34 im Bedienteil 5 mit einem dritten Bedienelement 35 in einer Wirkverbindung.

Das dritte Bedienelement 35 ist als ein um eine dritte Querachse 36, die parallel zur ersten Querachse 30 und zweiten Querachse 31 angeordnet ist, drehbares Rad 37 mit einem radial angeordneten Bedienhebel 38 ausgebildet. Das Rad 37 ist über eine seitlich und quer verlaufende Ausformung 39 mit dem proximalen Ende 34 der Hubstange 33 verbunden. Über eine Teildrehung des Rades 37 lässt sich über die Hubstange 33 der Gewebeanschlag 4 zwischen einer Grundstellung mit eingeklappten Backen 25, 26 und einer maximal ausgeklappten Stellung der Backen 25, 26 verstellen. End- und Zwischenstellungen sind dabei über eine zwischen dem dritten Bedienelement 35 und dem Bedienteil 5 angeordnete zweite Rastvorrichtung 40 fixierbar. Hierzu weist das Rad 37 an seinem Umfang Rastnuten 41 auf, in die ein federndes Rastteil 42 eingreifen kann. Damit ist der Gewebeanschlag 4 in unterschiedlichen Raststellungen bzw. Spreizstellungen verrastbar.

Die Backen 25, 26 bilden mit ihren in Aufklapprichtung außenliegenden Kanten die Anschlagflächen 47, 48, die in ihrer Wirkposition gegenüber der Längsachse 7 einen Ausklappwinkel 49 von beispielsweise 90° aufweisen und zur Anlenkstelle 19 einen fixen Abstand 50 von > 1,5 mm aufweisen. In den Ausführungsbeispielen beträgt der fixe Abstand 50 etwa 12 mm.

In den Ausführungsbeispielen ist die Schneide 3 als eine Elektrode ausgebildet, die mit einem Steckkontakt 43 am Bedienteil 5 elektrisch leitend verbunden ist. Der Steckkontakt 43 ist mit einer Steckerbuchse 44 eines Elektrodenkabels 45 verbindbar, das zu einem nicht dargestellten Elektrochirurgiegerät, einem Hochfrequenzgenerator, führt.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

Insbesondere kann der Gewebeanschlag 4, der gegenüber der Schneide 3 elektrisch isoliert ausgebildet ist, als eine zweite Elektrode verwendet werden, so dass der Gewebeanschlag 4 und die Schneide 3 ein bipolares Elektrodenpaar bildet, das mit dem nicht dargestellten Hochfrequenzgenerator bzw. elektrochirurgischen Gerät verbunden werden kann.

### Bezugszeichenliste

- 1: Instrument
- 2: Instrumentenschaft
- 3: Schneide
- 4: Gewebeanschlag
- 5: Bedienteil
- 6: distales Ende von 2
- 7: Längsachse von 2
- 8: Außenhülse
- 9: seitliche Öffnung von 8
- 10: proximales Ende von 2
- 11: Außenschaftrohr von 2
- 12: Innenschaftrohr von 2
- 13: Innenhülse
- 14: freies Ende von 3
- 15: Ausschwenkwinkel von 3
- 16: erste Längsseite von 3
- 17: zweite Längsseite von 3
- 18: Schneidkante
- 19: Anlenkstelle von 3
- 20: erstes Bedienelement
- 21: proximales Ende von 11
- 22: zweites Bedienelement
- 23: Hauptkörper von 5
- 24: Rastvorrichtung von 20, 22
- 25: erste Backe von 4
- 26: zweite Backe von 4
- 27: u-förmige Ausnahme
- 28: erste Führungsnut
- 29: zweite Führungsnut
- 30: erste Querachse von 27
- 31: zweite Querachse
- 32: distales Ende von 33
- 33: Hubstange
- 34: proximales Ende von 33
- 35: drittes Bedienelement
- 36: dritte Querachse
- 37: Rad von 35
- 38: Bedienhebel von 35
- 39: Ausformung
- 40: zweite Rastvorrichtung von 35
- 41: Rastnuten von 40
- 42: Rastteil von 40
- 43: Steckkontakt
- 44: Steckerbuchse
- 45: Elektrodenkabel
- 46: distal angelenktes Ende von 3
- 47: erste Anschlagfläche von 4
- 48: zweite Anschlagfläche
- 49: Ausklappwinkel von 47, 48
- 50: Abstand

## Patentansprüche

1. Instrument (1) zum Schneiden von Körpergewebe umfassend
- einen Instrumentenschaft (2),
- eine dem distalen Ende (6) des Instrumentenschaftes 82) benachbart angeordnete, gegenüber einer Längsachse (7) des Schaftes (2) ausklappbare und um die Längsachse (7) drehbare Schneide (3), und
- ein am proximalen Ende (10) des Instrumentenschaftes (2) angeordnetes Bedienteil (5),
wobei das distales Ende (6)des Instrumentenschaftes durch eine Trokarhülse in eine Körperhöhle mit zu schneidendem Gewebe einführbar ist,
wobei die Schneide (3) mit einem zum Bedienteil (5) hin gerichteten proximalen Ende (14) von der Längsachse weggerichtet um ihr dem proximalen Ende (14) abgewandtes und an einer Anlenkstelle (19) distal angelenktes Ende (46) seitlich ausschwenkbar ist,
und wobei am distalen Ende (6) des Instrumentenschaftes (2) ein Gewebeanschlag (4) angeordnet ist, und der Gewebeanschlag in eine Wirkposition bringbar ist, in der eine in proximaler Richtung zur Schneide (3) gewandte Anschlagfläche (47, 48) einen vorgegebenen fixen Abstand (50) zur Anlenkstelle (19) aufweist,
**dadurch gekennzeichnet,**
**dass** am distalen Ende (6) des Instrumentenschaftes (2) eine um die Längsachse (7) drehbare Außenhülse (8) angeordnet ist, die quer zu ihrer Längsrichtung eine seitliche Öffnung (9) aufweist, aus der die Schneide (3) ausschwenkbar ist.

2. Instrument nach Anspruch 1,
**dadurch gekennzeichnet,**
**dass** der fixe Abstand (50) größer als 1,5 mm ist.

3. Instrument nach Anspruch 1 oder 2,
**dadurch gekennzeichnet,**
**dass** der Gewebeanschlag (4) um eine am distalen Ende (6) des Instrumentenschaftes (2) angeordnete erste Querachse (30)in seine Wirkposition ausklappbar ist.

4. Instrument nach einem der Ansprüche 1 bis 3,
**dadurch gekennzeichnet,**
**dass** die Schneide (3) an mindestens einer ihrer beiden parallel zur Längsachse (7) verlaufenden Längsseiten (16, 17) eine Schneidkante (18) aufweist.

5. Instrument nach einem der Ansprüche 1 bis 4,
**dadurch gekennzeichnet,**
**dass** der Instrumentenschaft (2) ein Außenschaftrohr (11) aufweist, dessen distales Ende (6) mit der Außenhülse (8) verbunden ist,
**dass** in dem Außenschaftrohr (11) ein Innenschaftrohr (12) angeordnet ist,
**dass** zwischen Außenhülse (8) und Innenschaftrohr (12) eine an ihrer proximalen Stirnseite die Schneide (3) aufweisende Innenhülse (13) angeordnet ist,
**dass** die Innenhülse (13) zusammen mit der Außenhülse (8) und dem Außenschaftrohr (11) um das Innenschaftrohr (12) drehbar ist, und
**dass** das Außenschaftrohr (11) mit der Außenhülse (8) gegenüber dem Innenschaftrohr (12) und der Innenhülse (13) längsverschieblich ausgebildet sind.

6. Instrument nach Anspruch 5,
**dadurch gekennzeichnet,**
**dass** die Innenhülse (13) und die Schneide (3) mindestens im Bereich ihrer Anlenkstelle (19) aus einer Formgedächtnislegierung ausgebildet sind.

7. Instrument nach einem der Ansprüche 1 bis 6,
**dadurch gekennzeichnet,**
**dass** über ein am Bedienteil (5) angeordnetes längsverschiebliches erstes Bedienelement (20) die Außenhülse (8) über das Außenschaftrohr (11) zum Betätigen der Schneide (3) verschiebbar ist.

8. Instrument nach Anspruch 7,
**dadurch gekennzeichnet,**
**dass** das erste Bedienelement (20) in unterschiedlichen Raststellungen verrastbar ist.

9. Instrument nach Anspruch 7 oder 8,
**dadurch gekennzeichnet,**
**dass** über ein am Bedienteil (5) angeordnetes drehbares zweites Bedienelement (22) das Außenschaftrohr (11) mit der aus der Außenhülse (8) ausgeschwenkten Schneide (3) um die Längsachse (7) drehbar ist.

10. Instrument nach einem der Ansprüche 1 bis 9,
**dadurch gekennzeichnet,**
**dass** die Schneide (3) als eine Elektrode ausgebildet ist.

11. Instrument nach einem der Ansprüche 5 bis 10,
**dadurch gekennzeichnet,**
**dass** der Gewebeanschlag (4) am distalen Ende (6) des Instrumentenschaftes (2) zwei in einer u-förmigen Aufnahme (27) angeordnete Backen (25, 26) mit spiegelsymmetrisch ausgebildeten Führungsnuten (28, 29) aufweist,
**dass** die Backen (25, 26) mit ihren Führungsnuten (28, 29) entgegengesetzt zueinander um eine quer zur Längsachse (7) in der Aufnahme (27) angeordnete erste Querachse (30) verschwenkbar sind, und
**dass** die Backen (25, 26) an ihren proximalen Enden über eine zweite Querachse (31) mit einem distalen Ende (32) einer in dem Innenschaftrohr (12) längsverschieblich geführten Hubstange (33) verbunden sind.

12. Instrument nach Anspruch 11,
**dadurch gekennzeichnet,**
**dass** die Hubstange (33) an ihrem dem distalen Ende (32) abgewandten proximalen Ende (34) im Bedienteil (5) mit einem dritten Bedienelement (35) in Wirkverbindung steht.

13. Instrument nach Anspruch 12,
**dadurch gekennzeichnet,**
**dass** der Gewebeanschlag (4) über das dritte Bedienelement (35) in unterschiedlichen Raststellungen verrastbar ist.

14. Instrument nach einem der Ansprüche 1 bis 13,
**dadurch gekennzeichnet,**
**dass** der Gewebeanschlag (4) als eine Elektrode ausgebildet ist.

## Claims

1. An Instrument (1) for cutting body tissue, comprising
- an instrument shaft (2),
- a blade (3), which is arranged adjacent to the distal end (6) of the instrument shaft (2) and can be folded out with respect to a longitudinal axis (7) of the shaft (2) and can be rotated about the longitudinal axis (7), and
- an operating part (5) arranged at the proximal end (10) of the instrument shaft (2),
whereby the distal end (6) of the instrument shaft (2) can be inserted into a body cavity with tissue to be cut, through a trocar sleeve,
whereby a proximal end (14) of the blade (3) directed toward the operating part (5) can be laterally pivoted out away from the longitudinal axis about an end (46) of the blade that faces away from the proximal end (14) and that is distally articulated at an articulation point (19),
and whereby a tissue stop (4) is arranged at the distal end (6) of the instrument shaft (2), and the tissue stop can be brought into an active position in which a stop surface (47, 48) facing the blade (3) in the proximal direction has a specified fixed distance (50) from the articulation point (19),
**characterized in that**
an outer sleeve (8) rotatable about the longitudinal axis (7) is arranged at the distal end (6) of the instrument shaft (2), said outer sleeve having a lateral opening (9) transverse to its longitudinal direction, from which the blade (3) can be pivoted out.

2. The instrument according to Claim 1,
**characterized in that**
the fixed distance (50) is greater than 1.5mm.

3. The instrument according to Claim 1 or 2, **characterized in that**
the tissue stop (4) can be folded out into its active position about a first transverse axis (30) which is arranged at the distal end (6) of the instrument shaft (2).

4. The instrument according to any of Claims 1 to 3, **characterized in that**
the blade (3) has a cutting edge (18) on at least one of its two longitudinal sides (16, 17) that extend parallel to the longitudinal axis (7),

5. The instrument according to any of Claims 1 to 4,
**characterized in that**
the instrument shaft (2) has an outer shaft tube (11) whose distal end (6) is connected to the outer sleeve (8),
an inner shaft tube (12) is arranged in the outer shaft tube (11),
between the outer sleeve (8)and the inner shaft tube (12) an inner sleeve (13) is arranged with the blade (3) at its proximal end face,
the inner sleeve (13) together with the outer sleeve (8) and the outer shaft tube (11) is rotatable around the inner shaft tube (12), and
the outer shaft tube (11) with the outer sleeve (8) are designed to be longitudinally displaceable with respect to the inner shaft tube (12) and the inner sleeve (13).

6. The instrument according to Claim 5,
**characterized in that**
the inner sleeve (13) and the blade (3) are formed from a shape memory alloy at least in the region of their articulation point (19).

7. The instrument according to any of Claims 1 to 6,
**characterized in that**
by means of a longitudinally displaceable first operating element (20) arranged on the operating part (5) the outer sleeve (8) is movable over the outer shaft tube (11) to operate the blade (3).

8. The instrument according to Claim 7,
**characterized in that**
the first operating element (20) is lockable in different locking positions.

9. The instrument according to Claim 7 or 8,
**characterized in that**
by means of a rotatable second operating element (22) arranged on the operating part (5) the outer shaft tube (11) with the blade (3) pivoted out from the outer sleeve (8) is rotatable about the longitudinal axis (7).

10. The instrument according to any of Claims 1 to 9,
**characterized in that**
the blade (3) is formed as an electrode.

11. The instrument according to any of Claims 5 to 10,
**characterized in that**
the tissue stop (4) at the distal end (6) of the instrument shaft (2) has two jaws (25, 26) arranged in a U-shaped mounting bracket (27)with mirror-symmetrically formed guide grooves (28, 29),
the jaws (25, 26) with their guide grooves (28, 29) can be pivoted opposite to each other about a first transverse axis (30) arranged transversely to the longitudinal axis (7) in the mounting bracket (27), and the jaws (25, 26) are connected at their proximal ends via a second transverse axis (31) with a distal end (32) of a longitudinally displaceable lifting rod (33) located inside the inner shaft tube (12).

12. The instrument according to Claim 11,
**characterized in that**
the lifting rod (33) at its proximal end (34) facing away from the distal end (32) is operatively connected to a third operating element (35) in the operating part (5).

13. The instrument according to Claim 12,
**characterized in that**
by means of the third operating element (35) the tissue stop (4) is lockable in different locking positions.

14. The instrument according to any of Claims 1 to 13,
**characterized in that**
the tissue stop (4) is formed as an electrode.

## Revendications

1. Instrument (1) servant à couper des tissus corporels, comprenant
- une tige d'instrument (2),
- une lame (3) disposée au voisinage de l'extrémité distale (6) de la tige d'instrument (2), pouvant être basculée vers l'extérieur par rapport à un axe longitudinal (7) de la tige (2) et rotative autour de l'axe longitudinal (7),
- et une partie de manoeuvre (5) disposée à l'extrémité proximale (10) de la tige
d'instrument (2),
sachant que l'extrémité distale (6) de la tige d'instrument peut être introduite par un manchon de trocart dans une cavité corporelle pourvue de tissu à couper,
sachant que la lame (3) peut, par une extrémité proximale (14) dirigée vers la partie de manoeuvre (5), être pivotée latéralement vers l'extérieur en éloignement de l'axe longitudinal, autour de son extrémité (46) opposée à l'extrémité proximale (14) et articulée distalement à un point d'articulation (19), et sachant qu'une butée pour tissu (4) est disposée à l'extrémité distale (6) de la tige d'instrument (2), et la butée pour tissu peut être amenée dans une position opérationnelle dans laquelle une surface de butée (47, 48) tournée en direction proximale vers la lame (3) présente une distance fixe prédéterminée (50) par rapport au point d'articulation (19),
**caractérisé en ce qu**'un manchon extérieur (8) rotatif autour de l'axe longitudinal (7) est disposé à l'extrémité distale (6) de la tige d'instrument (2), manchon qui présente, transversalement à sa direction longitudinale, une ouverture latérale (9) par laquelle la lame (3) peut être pivotée vers l'extérieur.

2. Instrument selon la revendication 1, **caractérisé en ce que** la distance fixe (50) est supérieure à 1,5 mm.

3. Instrument selon la revendication 1 ou 2, **caractérisé en ce que** la butée pour tissu (4) peut être basculée vers l'extérieur dans sa position opérationnelle, autour d'un premier axe transversal (30) disposé à l'extrémité distale (6) de la tige d'instrument (2).

4. Instrument selon l'une des revendications 1 à 3, **caractérisé en ce que** la lame (3) présente un tranchant (18) sur au moins un de ses deux côtés longitudinaux (16, 17) s'étendant parallèlement à l'axe longitudinal (7).

5. Instrument selon l'une des revendications 1 à 4, **caractérisé en ce que** la tige d'instrument (2) présente un tube extérieur de tige (11), dont l'extrémité distale (6) est reliée au manchon extérieur (8),
**en ce qu'**un tube intérieur de tige (12) est disposé dans le tube extérieur de tige (11),
**en ce qu'**un manchon intérieur (13) présentant la lame (3) sur son côté frontal proximal est disposé entre le manchon extérieur (8) et le tube intérieur de tige (12),
**en ce que** le manchon intérieur (13) est rotatif autour du tube intérieur de tige (12) conjointement avec le manchon extérieur (8) et le tube extérieur de tige (11),
et **en ce que** le tube extérieur de tige (11) avec le manchon extérieur (8) est réalisé à coulissement longitudinal par rapport au tube intérieur de tige (12) et au manchon intérieur (13).

6. Instrument selon la revendication 5, **caractérisé en ce que** le manchon intérieur (13) et la lame (3) sont réalisés en un alliage à mémoire de forme au moins dans la région de leur point d'articulation (19).

7. Instrument selon l'une des revendications 1 à 6, **caractérisé en ce que** le manchon extérieur (8) peut être coulissé sur le tube extérieur de tige (11) au moyen d'un premier élément de manoeuvre (20) longitudinalement coulissant disposé sur la partie de manoeuvre (5), afin d'actionner la lame (3).

8. Instrument selon la revendication 7, **caractérisé en ce que** le premier élément de manoeuvre (20) peut être bloqué par enclenchement dans différentes positions d'enclenchement.

9. Instrument selon la revendication 7 ou 8, **caractérisé en ce que** le tube extérieur de tige (11), avec la lame (3) pivotée hors du manchon extérieur (8), est rotatif autour de l'axe longitudinal (7) au moyen d'un deuxième élément de manoeuvre rotatif (22) disposé sur la partie de manoeuvre (5).

10. Instrument selon l'une des revendications 1 à 9, **caractérisé en ce que** la lame (3) est réalisée sous la forme d'une électrode.

11. Instrument selon l'une des revendications 5 à 10, **caractérisé en ce que** la butée pour tissu (4) prévue à l'extrémité distale (6) de la tige d'instrument (2) présente deux mâchoires (25, 26) disposées dans un support (27) en forme de U et dotées de rainures de guidage (28, 29) réalisées symétriques,
**en ce que** les mâchoires (25, 26) avec leurs rainures de guidage (28, 29) peuvent être pivotées en opposition entre elles autour d'un premier axe transversal (30) disposé transversalement à l'axe longitudinal (7) dans le support (27),
et **en ce que** les mâchoires (25, 26) sont, à leurs extrémités proximales, reliées par l'intermédiaire d'un deuxième axe transversal (31) à une extrémité distale (32) d'une tige de levage (33) guidée en coulissement longitudinal dans le tube intérieur de tige (12).

12. Instrument selon la revendication 11, **caractérisé en ce que** la tige de levage (33) se trouve, à son extrémité proximale (34) opposée à l'extrémité distale (32), fonctionnellement reliée à un troisième élément de manoeuvre (35) dans la partie de manoeuvre (5).

13. Instrument selon la revendication 12, **caractérisé en ce que** la butée pour tissu (4) peut, au moyen du troisième élément de manoeuvre (35), être bloquée par enclenchement dans différentes positions d'enclenchement.

14. Instrument selon l'une des revendications 1 à 13, **caractérisé en ce que** la butée (4) pour le tissu est réalisée sous la forme d'une électrode.
